Europäisches Patentamt

European Patent Office

Office européen des brevets

(19)

(11) Publication number: **0 044 509**
**B1**

(12) **EUROPEAN PATENT SPECIFICATION**

(45) Date of publication of patent specification: **06.06.84**

(21) Application number: **81105519.3**

(22) Date of filing: **14.07.81**

(51) Int. Cl.³: **C 07 C 69/82,**
C 07 C 69/80, C 07 C 67/10
//B01J23/14

(54) Process for preparing diphenyl isophthalate and diphenyl terephthalate.

(30) Priority: **18.07.80 US 170993**

(43) Date of publication of application:
**27.01.82 Bulletin 82/4**

(45) Publication of the grant of the patent:
**06.06.84 Bulletin 84/23**

(84) Designated Contracting States:
**DE FR GB IT NL**

(56) References cited:
**GB - A - 999 947**
**US - A - 3 694 490**
**US - A - 3 705 186**
**US - A - 3 772 389**

(73) Proprietor: **GENERAL ELECTRIC COMPANY**
**1 River Road**
**Schenectady New York 12305 (US)**

(72) Inventor: **Fox, Daniel Wayne**
**193 Dawes Avenue**
**Pittsfield, Massachusetts 01201 (US)**
Inventor: **Kaduk, Bruce Alexander**
**57 Easton Avenue**
**Pittsfield, Massachusetts 01201 (US)**

(74) Representative: **Schüler, Horst, Dr. et al,**
**Kaiserstrasse 41**
**D-6000 Frankfurt/Main 1 (DE)**

## Description

### Background of the invention

Various methods have been disclosed for preparing diphenyl isophthalate and diphenyl terephthalate. In this connection, reference is made to the following patents: U.S. Patents 3,413,336 granted Nov. 26, 1968; No. 3,471,549 granted Oct. 7, 1969; No. 4,124,566 granted Nov. 7, 1978; and Canadian Patent 835,421 granted Feb. 24, 1970.

All of the above mentioned patents are based on reacting a phenolic compound, such as phenol with isophthalic, or terephthalic acid or alkyl esters thereof, together with another reagent to form the diphenyl esters of isophthalic and terephthalic acid. The required additional reagent is acetic anhydride (U.S. Patent Nos. 3,413,336 and 3,471,549 and Canadian Patent 835,421), or an aromatic hydrocarbon (U.S. Patent No. 4,124,566).

U.S. Patent 3,413,336 refers in its introduction broadly to a number of known processes which it regards as presenting various difficulties. In column 1, lines 55—61, it states: "It is also known to prepare aromatic dicarboxylic acid aryl esters by reacting the carboxylic acids with diaryl carbonates in the presence of catalysts. However, the process does not eliminate working with acid chlorides, since phosgene must be used in the preparation of the diarylcarbonates."

It is to be noted that no specific reagents (other than phosgene), proportions, conditions, catalyst, or specific process is disclosed.

The subject invention provides an improved and simplified process, which requires only a short reaction time, and which results in high yields of diphenyl isophthalate and diphenyl terephthalate in a purified state. The invention has a decided advantage over other processes used to produce diphenyl isophthalate and diphenyl terephthalate such as those which employ acid chlorides, those which employ phenyl acetates, and those which employ aromatic hydrocarbons such as ethyl benzene and xylene.

It has now been found that diphenyl isophthalate and diphenyl terephthalate can be readily prepared by heating isophthalic acid or terephthalic acid with at least a stoichiometric amount of diphenyl carbonate in the presence of from 0.25 to 0.50 mole percent stannous oxide (SnO) based on the isophthalic acid and terephthalic acid as a catalyst to a temperature of from 250°C to 300°C for a period from 30 minutes to 2.5 hours, depending on the temperature employed, removing the phenol formed in the resulting reaction during the heating period at atmospheric pressure, removing any excess diphenyl carbonate under vacuum, and recovering the diphenyl isophthalate or diphenyl terephthalate.

An especially preferred mole ratio of diphenyl carbonate employed in the subject process to the isophthalic acid and terephthalic acid will range from 6:1 to 3:1. Theory calls for a molar ratio of 2:1 and the process can be carried out at this ratio but yields and purity are lower than when ratio is more than 2:1.

Where vacuum is employed it is preferably at 1 mm pressure.

The removal of any excess diphenyl carbonate is preferably done by distillation at 1 mm at a temperature of 140—180°C., and the diphenyl isophthalate and diphenyl terephthalate are preferably recovered by distillation at 1 mm. at a temperature of 250—270°C.

More specifically in one of the preferred embodiments of the subject process, isophthalic acid or terephthalic acid (1 mole), diphenyl carbonate (3 moles), and stannous oxide (SnO) as the catalyst 0.25—0.50 mole percent based on the isophthalic or terephthalic acid) are charged into a reaction vessel equipped with an inert gas (for example, nitrogen) inlet, mechanical stirrer and distillation column. The mixture is heated to a temperature of 285—290°C for approximately two hours under a nitrogen sparge. During the heating period, phenol formed in the reaction is removed at atmospheric pressure, the Reaction is essentially complete within 1.5 hours. The mixture is then distilled at 1 mm. pressure at 140°C. for removal of excess diphenyl carbonate and at 1 mm. pressure at 250°C. to recover the diphenyl isophthalate and diphenyl terephthalate.

The subject invention is further described in the Examples which follow. These examples are preferred embodiments.

### Example 1

232 grams (1.08 moles) of diphenyl carbonate, 30 grams (0.18 mole) of isophthalic acid and SnO (0.12 grams, 0.5 mole percent based on the isophthalic acid) are charged into a 500 ml. flask equipped with a nitrogen inlet, mechanical stirrer, and distillation column. The mixture is heated to 280°C. for two hours under nitrogen sparge while removing the phenol formed in the reaction. During the heating period the phenol is removed at atmospheric pressure. The mixture is then distilled at 1 mm. pressure at 140°C. for removal of excess diphenyl carbonate and at 1 mm. pressure at 250°C. for recovery of diphenyl isophthalate.

### Example 2

232 grams (1.08) moles of diphenylcarbonate, 30 grams (0.18 mole) of terephthalic acid and SnO (0.12 grams, 0.5 mole percent based on the terephthalic acid) are charged into a flask as described and equipped in Example 1. The mixture is heated to 280—290°C. for two hours under nitrogen sparge while removing the phenol formed in the reaction. During the heating the phenol is removed under atmospheric pressure. The mixture is the distilled at 1 mm. pressure at 140°C. to remove excess diphenyl carbonate, and at 1 mm. pressure at 250°C. to recover the diphenyl terephthalate. Substantial formation of diphenyl terephthalate is already evident after heating for 30 minutes.

Example 3

464 grams (2.16 moles) of diphenyl carbonate, 60 grams (0.36 moles) of terephthalic acid, and SnO (0.24 grams, 0.5 mole percent based on the terephthalic acid) are charged into a 1-liter stainless steel kettle, equipped with a nitrogen inlet, a mechanical stirrer, and distillation column. The mixture is heated to 290—300°C., 2.5 hours under nitrogen sparge while removing the phenol formed in the reaction. During the heating period the phenol is removed at atmospheric pressure. The mixture is then distilled at 1 mm. at 140°C. for removal of excess diphenyl carbonate, and at 1 mm. pressure at 250°C. to recover diphenyl terephthalate in pure form.

Example 4

464 grams (2.16 moles) of diphenyl carbonate, 120 grams (0.72 mole) of isophthalic acid and SnO (0.24 gram, 0.25 mole percent based on the isophthalic acid) are charged into a kettle as described and equipped in Example 3. The mixture is heated to 290—300°C. for 2.5 hours under nitrogen sparge while removing the phenol formed in the reaction. During the heating period the phenol is removed at atmospheric pressure. The mixture is then distilled at 1 mm. pressure at 140°C. for removal of excess diphenyl carbonate, and at 1 mm. pressure at 250°C. to recover the diphenyl isophthalate.

Example 5

696 grams (3.25 moles) of diphenyl carbonate, 180 grams (1.08 moles) of isophthalic acid, and SnO (0.72 gram, 0.50 mole percent based on the isophthalic acid) are charged into a kettle as described and equipped as in Example 3. The mixture is heated to 290—300°C. for two hours under nitrogen sparge while removing the phenol formed in the reaction. During the heating period the phenol is removed under atmospheric pressure. After the phenol is removed, the contents are poured into a glass flask and the excess diphenyl carbonate is distilled off at 1 mm, pressure at 140—180°C., and distillation is further continued at 1 mm. pressure at 250—270°C. to recover the diphenyl isophthalate.

The foregoing detailed description and examples will suggest many variations to those skilled in this art. All such variations are within the full scope of the appended claims.

## Claims

1. A process of preparing diphenyl isophthalate and diphenyl terephthalate which comprises heating isophthalic acid or terephthalic acid with at least a stoichiometric amount of diphenyl carbonate in the presence of from 0.25 to 0.50 mole percent stannous oxide based on the isophthalic acid and terephthalic acid as a catalyst to a temperature of from 250°C to 300°C for a period from 30 minutes to 2.5 hours, removing the phenol formed in the resulting reaction during the heating period at atmospheric pressure, removing any excess diphenyl carbonate under vacuum, and recovering the diphenyl isophthalate and diphenyl terephthalate.

2. A process according to claim 1, where the molar ratio of diphenyl carbonate to the isophthalic acid and the terephthalic acid is from 6:1 to 3:1.

3. A process according to claim 1, wherein any excess diphenyl carbonate is distilled at 1 mm pressure at a temperature of 140°C to 180°C.

4. A process according to claim 1, wherein after removal of any excess diphenyl carbonate, the diphenyl isophthalate or the diphenyl terephthalate is recovered by distillation at 1 mm pressure at 250°C to 270°C.

5. A process according to claim 1, wherein isophthalic acid is heated with the diphenyl carbonate.

6. A process according to claim 1 wherein terephthalic acid is heated with the diphenyl carbonate.

7. A process according to claim 1, wherein the heating period is 2 hours and the heating is carried out under sparge of an inert gas.

8. A process according to claim 2, wherein the excess diphenyl carbonate is distilled at 1 mm pressure at a temperature of 140°C and the diphenyl isophthalate and diphenyl terephthalate recovered by distillation at 1 mm pressure at 250°C.

## Patentansprüche

1. Verfahren zur Herstellung von Diphenylisophthalat und Diphenylterephthalat, welches das Erhitzen von Isophthalsäure oder Terephthalsäure mit wenigstens einer stöchiometrischen Menge von Diphenylcarbonat in Anwesenheit von 0,25 bis 0,50 Mol-% Zinn (II)-Oxid, bezogen auf die Isophthalsäure und Terephthalsäure, als Katalysator auf eine Temperatur von 250 bis 300°C für die Dauer von 30 min. bis 2,5 h, das Entfernen des bei der Reaktion während der Erhitzungsperiode bei Atmosphärendruck gebildeten Phenols, das Entfernen von überschüssigem Diphenylcarbonat unter Vakuum und die Gewinnung des Diphenylisophthalats und Diphenylterephthalats umfaßt.

2. Verfahren nach Anspruch 1, bei dem das molare Verhältnis von Diphenylcarbonat zu der Isophthalsäure und der Terephthalsäure 6:1 bis 3:1 beträgt.

3. Verfahren nach Anspruch 1, bei dem überschüssiges Diphenylcarbonat bei einem Druck von 1 mm und einer Temperatur von 140°C bis 180°C abdestilliert wird.

4. Verfahren nach Anspruch 1, bei dem nach der Entfernung von überschüssigem Diphenylcarbonat das Diphenylisophthalat oder das Diphenylterephthalat durch Destillation bei einem Druck von 1 mm bei 250°C bis 270°C gewonnen wird.

5. Verfahren nach Anspruch 1, bei dem Isophthalsäure mit dem Diphenylcarbonat erhitzt wird.

6. Verfahren nach Anspruch 1, bei dem Terephthalsäure mit dem Diphenylcarbonat erhitzt wird.

7. Verfahren nach Anspruch 1, bei dem die Erhitzungsdauer 2 Std. beträgt und das Erhitzen unter dem Schutz eines Inertgases durchgeführt wird.

8. Verfahren nach Anspruch 2, bei dem das überschüssige Diphenylcarbonat bei einem Druck von 1 mm bei einer Temperatur von 140°C abdestilliert wird und das Diphenylisophthalat und das Diphenylterephthalat durch Destillation bei einem Druck von 1 mm bei 250°C gewonnen werden.

**Revendications**

1. Procédé de préparation de l'isophtalate de diphényle et du téréphtalate de diphényle qui comprend le chauffage d'acide isophtalique ou téfphtalique avec au moins une quantité stoéchiométrique de carbonate de diphényle en présence, comme catalyseur de 0,25 à 0,50 mole pourcent d'oxyde stanneux par rapport à l'acide isophtalique et à l'acide téréphtalique, à une température de 250°C à 300°C pendant de 30 minutes à 2,5 heures, l'élimination du phénol formé dans la réaction pendant le chauffage à la pression atmosphérique, l'élimination sous vide de tout excès de carbonate de diphényle, et la récupération de l'isophtalate de diphényle et du téréphtalate de diphényle.

2. Procédé selon la revendication 1, dans lequel le rapport molaire du carbonate de diphényle à l'acide isophtalique et à l'acide téréphtalique est de 6:1 à 3:1.

3. Procédé selon la revendication 1, dans lequel on distille tout excès de carbonate de diphényle sous 1 mm de pression à une température de 140°C à 180°C.

4. Procédé selon la revendication 1, dans lequel après l'enlèvement de tout excès de carbonate de diphényle, on récupère l'isophtalate ou le téréphtalate de diphényle par distillation sous 1 mm de pression à 250°C—270°C.

5. Procédé selon la revendication 1, dans lequel l'acide isophtalique est chauffé avec le carbonate de diphényle.

6. Procédé selon la revendication 1, dans lequel l'acide téréphtalique est chauffé avec le carbonate de diphényle.

7. Procédé selon la revendication 1, dans lequel la période de chauffage est de 2 heures et le chauffage s'effectue sous une nappe d'un gaz inerte.

8. Procédé selon la revendication 2, dans lequel on distille l'excès de carbonate de diphényle sous 1 mm de pression à une température de 140°C et on récupère l'isophtalate de diphényle et le téréphtalate de diphényle par distillation sous 1 mm de pression à 250°C.